# DEMANDE DE BREVET EUROPEEN

(11) **EP 0 524 899 A1**
(43) Date de publication de la demande: **27.01.1993**
(21) Numéro de dépôt: 92430015.5
(22) Date de dépôt: 30.04.1992
(51) Int. Cl.: C12N 15/12, C12N 15/79, C12P 21/08, A61K 39/395, G01N 33/577

(54) **Nouvelles molécules d'ADN recombinants contenant la partie codante d'une chaîne VB2 du récepteur pour l'antigène des cellules T, procédé de préparation, anticorps et médicaments les renfermant**

(30) Priorité: 02.05.1991 FR 9105712
(71) Demandeur: IMMUNOTECH S.A., F-13288 Marseille Cédex 9 (FR)
(72) Inventeur: Romagne, François, F-13008 Marseille (FR); Van Agthoven André, F-13009 Marseille (FR); Malissen, Bernard, F-13009 Marseille (FR); Besnardeau, Lydia, F-13008 Marseille (FR)
(74) Mandataire: Rinuy, Santarelli

(57) **Abrégé**

ADN recombinant comprenant une séquence nucléotidique chimère codant pour une chaîne Vβ2 du récepteur pour l'antigène des cellules T, ladite séquence étant constituée :
- d'une séquence provenant d'un humain et codant pour la totalité de la partie variable (Vβ2), diversité (Dβ) et de jonction (Jβ), et
- d'une séquence d'origine murine codant pour la partie complémentaire constante (Cβ2) absente de la séquence humaine ci-dessus,

les deux séquences étant liées au niveau d'un site de restriction naturel ou créé artificiellement,
anticorps obtenus par utilisation desdits ADN,
compositions pharmaceutiques, produits de diagnostic les renfermant, et procédé de préparation.

## Description

La présente invention concerne de nouvelles molécules d' ADN recombinants codant notamment pour une chaîne humaine Vβ2 du récepteur pour l'antigène des cellules T, procédé de préparation, anticorps et médicaments les renfermant.

Il serait intéressant de développer facilement un panel d'anticorps dirigé contre les différentes chaînes de récepteurs pour l'antigène des cellules T (ci-après TCR) humaines , afin par exemple d'obtenir une discrimination et une numération précise entre les différentes sous-populations de cellules T humaines.

L'étude du déséquilibre entre les sous-populations permettrait le diagnostic différentiel des maladies provoquant une prolifération de cellules T. Elle pourrait aussi être le témoin d'une réponse immunitaire spécifique contre un tissu particulier, pathogène tel qu'une tumeur par exemple. On sait en particulier que des anticorps contre les antigènes CD₄ et CD₈ qui distinguent deux populations de cellules T, sont témoins d'une série de maladies immunitaires dont le SIDA.

Ces anticorps devraient pouvoir être réalisés facilement et en grande quantité.

On sait aussi que la toxine TSST1 des staphylocoques active spécifiquement les lymphocytes T humains porteurs de chaînes Vβ2. Il serait donc intéressant de disposer d'anticorps anti Vβ2 afin de permettre la détection et la numération in vivo et in vitro des cellules T porteuses de chaînes Vβ2. Il serait aussi très intéressant de disposer d'un traitement curatif et/ou préventif du choc endotoxinique, notamment dû aux staphylocoques.

La demanderesse a découvert de nouveaux ADN recombinants permettant la préparation de nouveaux vecteurs d'expression eucaryote qui étaient de remarquables agents de transfection de cellules eucaryotes, lesquelles cellules transfectées étaient d'excellents immunogènes conduisant à des anticorps, notamment monoclonaux, dirigés vers la chaîne Vβ2 des TCR humains.

C'est pourquoi la présente demande à pour objet un ADN recombinant caractérisé en ce qu'il comprend une séquence nucléotidique chimère codant pour une chaîne du récepteur pour l'antigène des cellules T, ladite séquence étant constituée :
- d'une séquence d'origine humaine et codant pour la totalité de la partie variable Vβ2 et de préférence sa séquence de tête, diversité Dβ et de jonction Jβ,
- et d'une séquence d'origine murine codant pour la partie complémentaire constante (Cβ1 ou Cβ2) absente de la séquence humaine ci-dessus,

les deux séquences étant liées au niveau d'un site de restriction naturel ou créé artificiellement. Dans ce qui suit, on appellera ce site "site sélectionné".

Par ADN recombinant l'on entend une séquence de nucléotides artificiellement crée.

Par chimère l'on entend que l'ADN recombinant comprend au moins deux séquences provenant d'origines différentes, par exemple 3, 4 ou 5 et de préférence 2 séquences de deux origines différentes.

Ces séquences proviennent de deux espèces différentes et l'origine de l'une d'entre elles est murine; l'autre (ou les autres) proviendra de l'homme.

Le récepteur αβ des cellules T, est une protéine hétérodimère composée de deux chaînes polypeptidiques reliées par un pont disulfure. Le récepteur est exprimé à la surface des cellules T en association avec le complexe CD3. Le locus correspondant à une chaîne particulière du TCR comprend une importante sélection de segments géniques distincts, incluant de nombreux segments V différents, plusieurs segments J et un ou deux segments constants C. Ces segments peuvent subir des réarrangements somatiques pendant la maturation thymique des cellules T produisant ainsi un gène unique, qui est transcrit et contenant des segments V, des segments J, un segment C et, le cas échéant, un segment D entre les segments V et J.

Dans l'ADN recombinant chimère ci-dessus décrit, la totalité de la séquence codant pour la partie Cβ peut provenir d'un murin et dans ce cas la séquence humaine ne comprend aucun nucléotide correspondant à la partie Cβ. De préférence, la séquence d'origine humaine comprend une courte partie Cβ, de préférence inférieure à 100 nucléotides et notamment environ 60 nucléotides ; de ce fait, seule une large partie Cβ de la chimère provient du murin (cette partie étant la partie complémentaire absente de la séquence humaine ci-dessus) et le reste étant d'origine humaine. Les deux séquences d'origines différentes sont liées au niveau d'un site de restriction sélectionné, en phase dans le cadre de lecture.

La séquence humaine comprendra nécessairement en plus des parties Vβ2, Jβ et éventuellement Dβ et une partie de Cβ, la totalité de la séquence de tête.
Le site de restriction sélectionné peut être naturellement présent sur chacune des deux séquences ou présent naturellement sur une seule des deux séquences, ledit site de restriction étant artificiellement créé sur l'autre séquence.Le site de restriction ci-dessus doit être unique sur le segment Cβ2 murin et de préférence unique sur le segment d'origine humaine.

Lorsque le site de restriction sélectionné est créé artificiellement, il peut être réalisé par exemple par délétion ou adjonction de nucléotides ou de préférence par mutation selon les méthodes connues en elles-mêmes, notamment par mutagénèse dirigée. L'altération ainsi réalisée de l'ADN permet l'introduction d'un site de restriction normalement absent. En particulier, un site BglII, présent naturellement au niveau de l'ADN codant pour la région Cβ1 et Cβ2 humaine, mais absent du gène codant pour la chaîne du TCR de la souris, peut être introduit dans le gène Cβ2 de la souris par mutagénèse dirigée, mais éventuellement mutagénèse par polymérisation en chaîne (PCR). La mutation permet de conserver le maximum d'homologie avec la ou les séquences de départ. Le site de restriction ainsi créé sera de préférence placé au niveau d'un site homologue au site au niveau duquel le site de restriction est naturellement présent chez le mammifère considéré, l'homme, de manière à permettre la coupure, tant de la séquence murine que de la séquence humaine à l'aide de ladite enzyme, pour ensuite, par ligation en phase dans le cadre de lecture, créer la chimère désirée. De ce qui précède, on comprend que si l'on choisit une enzyme de restriction donnée, un seul site de restriction correspondant doit au plus être présent sur le segment Cβ2 murin et de préférence unique sur le segment d'origine humaine et de plus, au niveau de la séquence codant pour la partie Cβ2, à un emplacement permettant la transcription en phase d'un ADN codant notamment la chaîne du TCR.

Des ADN recombinants préférés selon l'invention sont caractérisés en ce que le site de restriction est naturel sur la séquence codant pour la partie C humaine.

Des chimères tout particulièrement préférées selon l'invention ont une partie humaine caractérisée en ce que sa séquence d'origine humaine est la séquence codant pour des segments Vβ2, Dβ, Jβ humains, et une courte partie Cβ jusqu'au site unique BglII de Cβ.

La partie murine des chimères notables selon l'invention est caractérisée en ce que le site de restriction, notamment BglII est artificiellement créé sur la chaîne d'origine murine.

L'ADN recombinant selon l'invention peut se présenter sous forme d'une séquence nucléotidique mono ou simple brin, limitée aux parties V, D, J et C, ci-dessus, ou comportant d'autres nucléotides, par exemple et notamment ceux constituant la séquence de tête.

C'est ainsi que la séquence ci-dessus peut en particulier être incluse dans un vecteur d'expression, et dans ce cas de préférence dans un vecteur d'expression eucaryote.

La séquence ci-dessus peut également être incluse en vue de sa réplication dans un autre vecteur, par exemple dans un plasmide tel que par exemple BLUESCRIPT.

Comme vecteur d'expression eucaryote on peut citer tout particulièrement pHβPr1-Néo. On peut citer également pHβPr1gpt et généralement tout vecteur d'expression eucaryote possédant les signaux de régulation promoteur et un signal de polyadénylation permettant l'expression de l'ADNc. Bien évidemment, le choix du couple vecteur-hôte est de la compétence de l'homme de l'art. Des ADN recombinants tout particulièrement préférés selon l'invention sont caractérisés en ce qu'ils se présentent sous forme d'un vecteur d'expression eucaryote, lequel étant de préférence dérivé de pHβPr1-Néo.

L'ADN recombinant ci-dessus, notamment sous forme de vecteur d'expression eucaryote, peut être utilisé pour transfecter dans un hôte approprié la séquence codant pour le TCR. Des cellules hôtes ainsi transfectées, éventuellement traitées, par exemple préalablement transfectées avec la chaîne CD3 Zeta pour obtenir une meilleure expression, peuvent être utilisées comme immunogène de préférence chez la souris.

On peut ainsi classiquement préparer des anticorps, notamment monoclonaux, le criblage des hybridomes obtenus à partir de cellules de myélome et de cellules de rate du murin utilisé, pouvant se faire par la reconnaissance de la lignée transfectée, versus la non-reconnaissance de la lignée de départ.

C'est pourquoi, la présente demande a également pour objet les anticorps, notamment monoclonaux, caractérisés en ce qu'ils sont préparés par mise en oeuvre d'un ADN recombinant ci-dessus décrit.

La présente invention a aussi pour objet les anticorps anti chaîne Vβ2 du récepteur de cellules T. Les anticorps ci-dessus présentent de remarquables propriétés car ils sont parfaitement caractérisés vis à vis des chaînes β de TCR, et particulièrement vis à vis des régions variables Vβ2 des chaînes de TCR, mais aussi vis-à-vis des régions Dβ et Jβ dans leur réarrangement typique.

Ces anticorps sont donc capables de distinguer les éléments Vβ2 de la population des cellules T humaines et ainsi spécifiquement reconnaître, par exemple une maladie ou une déficience donnée.

Dans les anticorps ci-dessus décrits on préfère les anticorps anti partie variable, notamment Vβ2, qui présentent de remarquables propriétés à la fois pour le diagnostic et la thérapie, curative ou préventive, chez l'homme ou l'animal, des maladies auto-immunes et des maladies septiques, notamment celles dues aux staphylocoques, particulièrement ceux à toxine TSST1.

Les anticorps selon l'invention peuvent à cette fin être utilisés directement ou, si désiré, couplés selon les méthodes connues en elles-mêmes, par exemple à l'aide d'un agent hétérobifonctionnel comme le SMCC, à une toxine ou un cytotoxique auxquels sont sensibles les cellules T, réduisant ainsi notamment les réactions du choc septique.

C'est pourquoi la présente invention a aussi pour objet les compositions pharmaceutiques caractérisées en ce qu'elles renferment au moins un des anticorps tels que définis ci-dessus.

Les compositions pharmaceutiques peuvent se présenter notamment sous les formes parentérales, notamment injectables, couramment utilisées en médecine humaine, par exemple pour la voie sous cutanée, intramusculaire ou intraveineuse.

Les anticorps ci-dessus peuvent également être utilisés pour soigner les maladies immunes ou provoquant une prolifération des cellules T porteuses de chaînes Vβ2, ainsi que les leucémies à cellules T porteuses de chaîne Vβ2 par injection des anticorps ci-dessus.

Les anticorps peuvent aussi être couplés à des produits radioactifs, selon des méthodes connues en elles-mêmes (Chelatants de métaux comme l'Indium, substitution d'Iode, etc ...).

Les anticorps selon l'invention trouvent leur emploi notamment dans le traitement du choc septique dû aux staphylocoques et des maladies auto-immunes telles que l'arthrite rhumatoïde, le diabète, la maladie de Sjögren ou le lupus érythémateux.

La dose usuelle, variable selon le produit utilisé, le sujet traité et l'affection en cause, peut être par exemple de 0,001 à 25 mg par jour et par kg de poids corporel chez l'homme d'un anticorps monoclonal selon l'invention couplé à la ricine A à titre de cytotoxique, et de préférence de 0,05 à 0,2 mg/kg/jour.

Etant donné que des anticorps, notamment monoclonaux, ci-dessus trouvent aussi leur utilisation à des fins diagnostiques, pour la détection, l'identification ou le dosage des TCR ou de parties de TCR à chaînes Vβ2, la présente invention a également pour objet l'utilisation desdits anticorps ainsi que les compositions diagnostiques destinées à la détection, l'identification ou le dosage des antigènes ci-dessus.

Les anticorps ci-dessus trouvent également leur utilisation dans la purification des produits renfermant la partie antigénique correspondante à des chaînes de TCR. Les anticorps ci-dessus peuvent être utilisés à fins diagnostiques en cytofluorimétrie et dans ce but peuvent être couplés, par des méthodes connues en elles-mêmes, à des marqueurs fluorescents tels que l'isothiocyanate de fluorescéine (FITC) ou la phycoérythrine, technique plus avantageuse que l'étude par PCR.

On peut, par exemple, détecter la présence d'une population de cellules T portant la chaîne Vβ2 en détectant le segment variable Vβ2 au niveau des tissus souffrant par exemple d'une infection, d'un cancer ou d'une maladie auto-immune telle la sarcoïdose, et en détectant sa présence selon des méthodes connues en elles-mêmes.

On peut également marquer les anticorps ci-dessus décrits, à l'aide d'enzymes. On peut citer par exemple, la glucose 6 phosphate deshydrogénase, l'acétyl-cholinéstérase, la glucoseoxydase ou la bétagalactosidase. Les anticorps totaux ci-dessus peuvent également être couplés à un radiomarqueur tel que l'indium 111, pour utilisation dans l'imagerie des populations de cellules T comportant la région Vβ2, au niveau des parties malades du corps humain.

La présente invention a également pour objet un procédé de préparation des ADN recombinants ci-dessus décrits, caractérisé en ce qu'on ligue :
- une séquence nucléotidique constituée d'au moins la totalité de la séquence codant pour les parties Vβ2, Dβ et Jβ d'une chaîne du récepteur de l'antigène des cellules T humain jusqu'au niveau d'un site de restriction, avec
- une séquence nucléotidique d'origine murine comprenant l'homologue de la partie complémentaire de la partie Cβ2 absente de la séquence de chaîne humaine ci-dessus, à partir du même site de restriction que ci-dessus, ledit site de restriction étant artificiellement créé sur l'une, l'autre ou les deux séquences, pour obtenir la chimère attendue, puis si désiré, ligue une séquence comprenant la totalité des parties Vβ2, Dβ, Jβ et Cβ ci-dessus avec un vecteur d'expression eucaryote, puis si désiré, isole la nouvelle chimère obtenue.

Dans des conditions préférentielles, le procédé décrit ci-dessus, est caractérisé en ce qu'en outre l'on transfecte une cellule eucaryote comportant tous les éléments permettant la synthèse du récepteur de l'antigène des cellules T, à l'exception de la chaîne homologue à la chaîne codée par la séquence chimère ci-dessus, utilise comme antigène les cellules ainsi transfectées chez la souche murine correspondante, fusionne des cellules de rate prélevées sur les murins auxquels a été administré l'antigène à des cellules de myélome pour préparer, selon les méthodes connues en elles-mêmes, des anticorps dirigés contre le produit de la séquence variable humaine.

Le procédé ci-dessus décrit peut notamment être réalisé comme suit :
La séquence nucléotidique codant pour les parties Vβ2, Dβ et Jβ d'une chaîne du TCR humain est notamment un ADN double brin linéaire comportant la séquence codant pour les parties Vβ2, Jβ, Dβ2 et éventuellement Cβ en partie, d'une chaîne de TCR humain, de même que son gène de tête, est obtenue par coupure à l'aide de deux enzymes de restriction différentes : l'enzyme correspondant au site de restriction sélectionné au niveau de la partie Cβ, par exemple BglII et une autre enzyme clivant la séquence du côté opposé, après le gène de tête, par exemple EcoRI à partir d'une séquence d'ADN double brin linéaire comportant ladite séquence. L'ADN ainsi obtenu est ligué à un vecteur traité par les mêmes deux enzymes de restriction, ce vecteur comportant la séquence codant pour le segment Cβ2 de la chaîne de TCR murin, et les mêmes sites de restriction que ci-dessus, par exemple BglII et EcoRI . On obtient ainsi le gène chimère attendu. Celui-ci n'est toutefois pas exprimable tel quel. C'est pourquoi d'urne part le vecteur comportant la chimère est traité par deux enzymes de restriction coupant de part et d'autre du gène chimère codant pour le TCR chimère ; d'autre part, on traite par les mêmes deux enzymes de restriction un vecteur d'expression eucaryote. Par ligation du gène obtenu ci-dessus codant pour le TCR chimère avec ledit vecteur d'expression eucaryote, on obtient un nouveau vecteur chimère d'expression eucaryote. Ledit vecteur d'expression eucaryote doit comporter les éléments nécessaires pour assurer la transcription correcte du gène chimère codant pour le TCR. A cet égard, on peut citer par exemple comme vecteur pHβPr1-Néo, qui peut être scindé par BamHI et SalI, et comprend notamment le gène de résistance à la néomycine utilisé comme un marqueur de sélection, un gène promoteur eucaryote SV40, ainsi qu'un signal polyadenylation à proximité du site BamHI. Le nouveau vecteur chimère obtenu est avantageusement utilisé pour transfecter des cellules eucaryotes, de préférence des cellules T de souris, notamment des lignées cellulaires comportant tous les éléments pour l'expression du TCR à l'exception du gène apporté par le vecteur chimère. On peut citer par exemple, la lignée de souris DOIS19 ou celle du nom de DS 23.27.4. De telles cellules permettent une sélection facile des cellules exprimant la chimère qui seules sont capables d'exprimer le TCR à la surface de la cellule.

Bien entendu, chaque ligation (ci-dessus et ci-après) est suivie de la transfection d'un hôte approprié et la sélection des cellules contenant la chimère désirée. Les cellules transfectées peuvent être administrées à titre d'antigène de préférence à des souris, par exemple par injection intrapéritonéale. On peut ainsi prélever des cellules de rate des murins ainsi traitées, cellules capables de sécréter les anticorps recherchés pour les fusionner à des cellules immortelles, telles que le myélome. Les hybridomes sélectionnés produisant les anticorps recherchés permettent ainsi la préparation d'anticorps notamment monoclomaux dirigés contre la chaîne Vβ2 du TCR du mammifère choisi, notamment l'homme.

L'ADN double brin codant pour la chaîne du récepteur de l'antigène des cellules T humain peut par exemple être préparé comme suit :
L'étude de la séquence des gènes codant pour la chaîne Vβ2 permet de sélectionner un site de restriction déjà présent sur une partie constante Cβ, soit alors amène à créer sur ladite séquence un tel site et permet la synthèse de nouveaux oligonucléotides utilisables pour synthétiser l'ADN complémentaire à partir de l'ARN messager.

On purifie à partir de lymphocytes T humains l'ARNm codant pour une chaîne TCR donnée. On synthétise l'ADN complémentaire à l'aide d'un oligonucléotide, par exemple OHCβ, spécifique d'un gène humain donné et contenant le cas échéant le site unique de restriction sélectionné à l'aide d'une transcriptase reverse, synthétise le deuxième brin à partir d'un oligonucléotide spécifique de la chaîne, par exemple OL2, à l'aide d'une polymérase, et amplifie le gène obtenu par réactions de polymérisation en chaîne (PCR) en effectuant par exemple une trentaine de cycles.

On obtient ainsi une grande quantité d'ADN double brin linéaire codant. On peut alors couper par les enzymes appropriées, par exemple BglII et EcoRI, les fragments produits par PCR, de manière à éliminer tout ou partie de la séquence codant pour la partie Cβ, à partir du site sélectionné et de manière à conserver les séquences Vβ2, Dβ et Jβ, ainsi que la totalité de la séquence de tête.
- La séquence nucléotidique d'origine murine comprenant la partie complémentaire de la partie Cβ2 absente de la séquence de chaîne humaine ci-dessus, dans le cas où celle-ci se trouve sous la forme d'un vecteur comportant ladite séquence, peut être préparée comme suit :

On isole un ADNc complet contenant toute la partie codante de la chaîne β du TCR choisi à partir d'une banque d'ADNc provenant d'une lignée cellulaire murine, par exemple KB5C20, à l'aide d'une sonde connue ; on clone cet ADNc dans un plasmide approprié, par exemple PUC19, puis on transfecte le plasmide résultant appelé dans ce cas pUCmCβ2 dans un hôte, par exemple la souche bactérienne DH1 de E. coli.

On pourra ensuite cloner le fragment intéressant d'ADN murin dans un phage. Pour cela, on peut digérer pUCmCβ2 par des enzymes de restriction appropriées, par exemple EcoRI et HindIII, de manière à obtenir trois fragments dans le cas ci-dessus. On purifie le fragment contenant toute la séquence codante de la chaîne Cβ2 murine désirée (dans le cas des enzymes précitées, EcoRI coupe dans la partie V de la séquence). On obtient ainsi une séquence codant pour une partie de la partie V, ainsi que la totalité des parties Dβ, Jβ et Cβ. On ligue alors ladite séquence avec un phage approprié, par exemple M13mp18, ouvert avec les mêmes enzymes de restriction. Dans ce cas, l'ADN phagique existe sous deux formes : simple brin pour le phage encapsidé et double brin pour sa forme réplicative. On hybride un oligonucléotide complémentaire de la région à muter, sauf pour la mutation désirée, avec la forme simple brin du phage. Le brin complémentaire est synthétisé in vitro par une ADN polymérase utilisant le même oligonucléotide pour initier la réaction. On ferme le nouveau brin sur lui-même à l'aide d'une ligase. Le double brin complètement homologue est alors transfecté par exemple dans E. coli, notamment JM101 Les colonies mutantes sont sélectionnées, par exemple par hybridation avec un oligonucléotide radio-marqué.

Pour des détails sur la mutagénèse dirigée, se référer à MANIATIS et al ( Molecular Cloning ; Elaboratory Manual, 1989, Cold Spring Harbour Laboratory Press).

On purifie l'ADN double brin d'un sous-clone comportant la mutation désirée et dont le reste de la séquence codant pour la partie Cβ2 est resté intègre, et le coupe par des enzymes appropriées, par exemple EcoRI et EcoRV pour isoler le fragment renfermant le gène désiré. On ligue alors ce fragment dans un plasmide adapté, qu'il soit possible de séquencer et comportant des sites de restriction permettant d'extraire facilement la séquence insérée pour l'introduire dans un vecteur d'expression et préalablement ouvert par des enzymes compatibles présentes dans son site de liaisons multiples, par exemple par EcoRI et SmaI. Comme à l'accoutumée,le produit de la ligation peut alors être transfecté dans un hôte approprié, par exemple une souche bactérienne E. coli DH1. En analysant, par exemple, par coupure avec deux couples d'enzymes tels que EcoRI-XBaI et EcoRI-BglII, on peut sélectionner un clone fournissant en électrophorèse les bandes attendues.

Le plasmide purifié comprend la séquence nucléotidique d'origine murine attendue complémentaire de la partie C absente de la séquence de chaîne humaine ci-dessus à partir du même site de restriction de manière à permettre la ligation en phase dans le cadre lecture.

Les différentes techniques utilisées ci-dessus sont bien connues de l'homme de l'art et ont fait l'objet de nombreuses publications faisant partie de l'état de la technique.

Les exemples qui suivent illustrent la présente invention sans toutefois la limiter.

La Figure 1 représente les comparaisons des ADNc de chaînes Cβ2 de souris et Cβ1 humaines de TCR.

La Figure 2 représente un oligonucléotide complémentaire de l'ADNc codant pour la chaîne Cβ2 du TCR murin sauf au niveau des deux bases nécessaires pour introduire le site BglII.

La figure 3 représente un oligonucléotide spécifique des chaînes Vβ2 de TCR humain.

La figure 4 représente les profils de cytométrie de flux obtenus avec un anticorps anti CD3 de souris de transfectants de DOIS19 (profils noirs) obtenus avec deux ADN chimériques de l'invention contenant un Vβ2 humain, mais de régions D et J différentes (histogramme de gauche FRN2.7.6 histogramme de droite FRN2.11.4) par rapport à la lignée de départ DOIS19 (profils blancs).

La figure 5 montre l'analyse de deux transfectants Vβ2 (profils noirs) : en haut FRN2.7.6 ; en bas FRN2.11.2 par le surnageant de E25G8 (histogrammes de droite) et le monoclonal anti CD3 murin (histogrammes de gauche) par rapport à la lignée de départ DOIS19 (profils blancs).

La figure 6 montre l'analyse de deux transfectants Vβ2 (profils noirs) à gauche FRN2.7.6 ; à droite FRN2.11.2 par le surnageant E22E7.2 (histogrammes du bas) et le monoclonal anti CD3 murin (histogrammes du haut) par rapport à la lignée de départ DOIS19 (profils blancs).

La figure 7 représente le marquage en double couleur (anti CD3 humain d'Immunotech marqué à la phicoérythrine : E22E7. 2 révélé par GAMIG-FITC) des lymphocytes de sang périphérique.

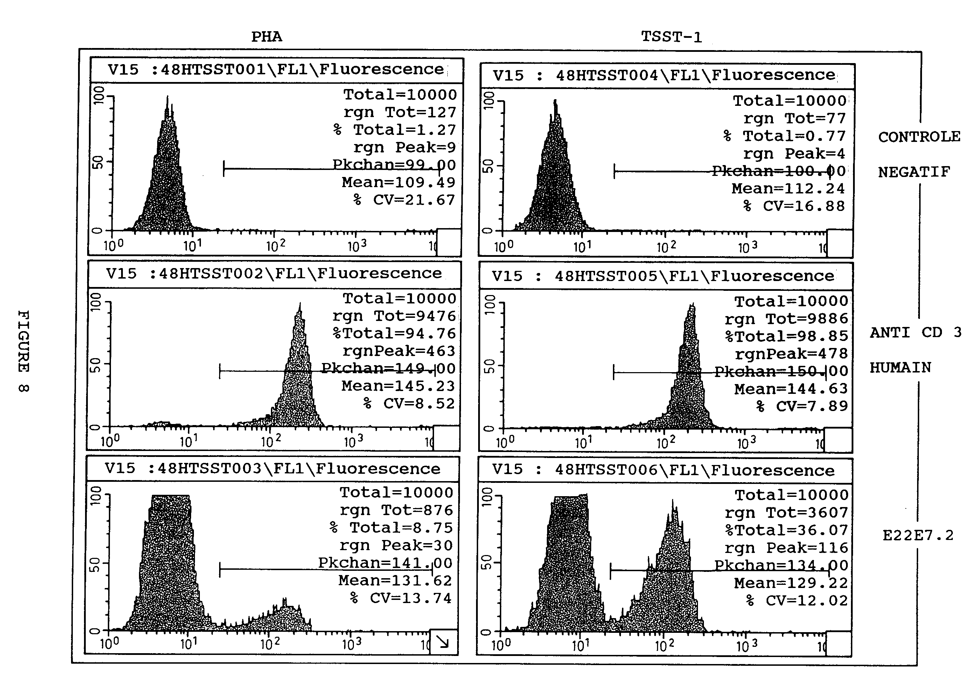

La figure 8 montre l'expansion spécifique du compartiment Vβ2 positif après stimulation de lymphocytes T périphériques par la TSST (histogrammes de droite) par rapport à la PHA (histogrammes de gauche).

### PARTIE EXPERIMENTALE

### EXEMPLE 1

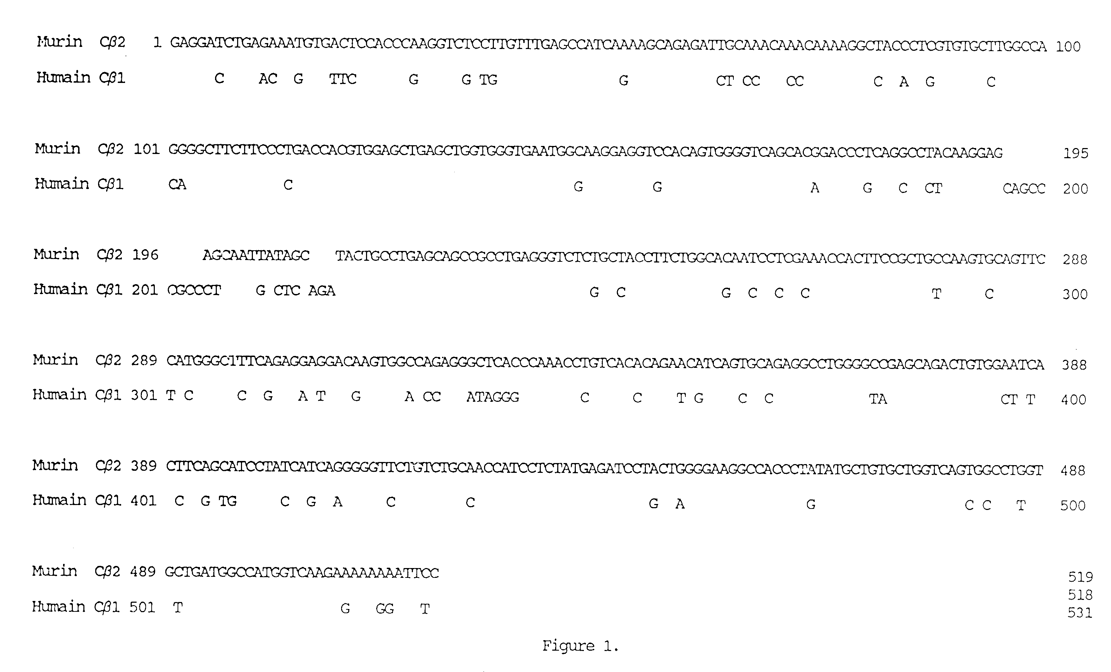

On tire parti de l'existence d'un site BglII sur le gène codant pour les chaînes β1 et β2 du TCR humain dans leur région C, à proximité de la région J, et de celle d'un site identique à l'exception de deux bases dans la partie homologue de la souris (cf. Figure 1).
A) Préparation de la séquence nucléotidique codant pour la chaîne Cβ2 murine mutée contenant un site BglII dans la séquence homologue correspondant au site BglII présent sur les chaînes Cβ1 et Cβ2 humaines sous forme d'un vecteur comportant ladite séquence.

### 1. Chaîne Cβ2 murine

La séquence de la chaîne Cβ2 murine est décrite en détail dans Malissen M. et al (1984), Cell 37 : 1101-1110. Un ADNc complet contenant toute la partie codante de Cβ2 a été obtenu à partir d'une banque d'ADNc de la lignée cellulaire murine KB5C20 (décrite dans Albert et al (1982), Immunogenetics 16 : 533-549). Cet ADNc a été cloné dans le plasmide pUC19. Le plasmide résultant pUCmCβ2 a été transfecté dans la souche bactérienne DH1. La bactérie recombinante a été déposée auprès de la Collection Nationale de Cultures de Microorganismes (CNCM) à Paris le 12 Février 1991 sous le n° I-1032 et disponible sans restriction.

### 2. Mutagénèse dirigée de l'ADNc codant la chaîne Cβ2 murine.

Principe : La procédure de mutagénèse dirigée choisie utilise le phage filamenteux M13mp18 BIORAD. Ce bactériophage est décrit précisément dans Norrander et al (1983) Gene 26 : 101-106. Il est disponible commercialement chez plusieurs fabricants, notamment BIORAD. Le principe de la manipulation est le suivant :
on clone le fragment d'ADN à modifier dans le site de liaisons multiples du phage. Ce vecteur phagique existe sous deux formes au cours de son cycle de vie. La forme réplicative, dans la bactérie, est essentiellement double brin, la forme encapsidée est simple brin. L'ADN des deux formes est purifiable. Un oligonucléotide complémentaire de la région à muter, sauf pour la mutation désirée, est hybridé avec la forme simple brin du phage. Le brin complémentaire est ensuite synthétisé in vitro par une ADN polymérase, utilisant l'oligonucléotide comportant la mutation souhaitée pour initier la réaction. Une ligase est utilisée pour fermer le nouveau brin sur lui- même, à l'extrémité 5′ de l'oligonucléotide. Le double brin complètement homologue, sauf pour la mutation voulue, est transfecté dans E. Coli résultant en deux classes de colonies mutantes et non mutantes. Les colonies mutantes sont ensuite sélectionnées.

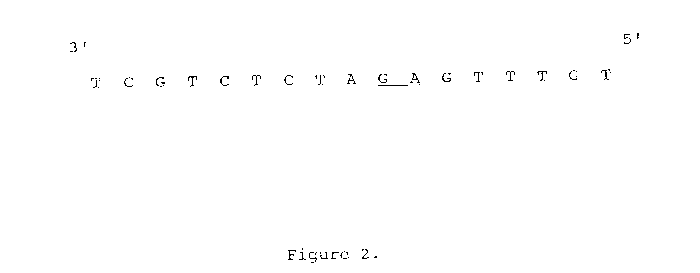

Pour introduire le site BglII dans l'ADNc de la chaîne Cβ2 de souris, on clone cet ADNc dans le phage M13mp18. On utilise l'oligonucléotide de la figure 2 pour faire la mutagénèse. Cet oligonucléotide est complémentaire de l'ADNc codant pour le gène Cβ2 murin sauf pour les deux bases nécessaires pour introduire le site BglII. Ces deux bases sont soulignées sur la figure 2.

Les procédures de clonage, transfection, étalement, analyses des colonies, purification des formes simples et doubles brins pour le phage M13mp18 sont décrites précisément par Maniatis et al (déjà cité). L'hôte bactérien utilisé est la souche JM101 le E. coli.

### Expérimentation :

### a) Clonage de Cβ2 murin dans M13mp18

On digère pUCmCβ2 par Eco RI et Hind III. Cette digestion fournit 3 fragments distinguables par électrophorèse d'Agarose. On purifie le fragment intermédiaire de 1000 paires de bases (pb) environ contenant toute la séquence codante de Cβ2 murine. Ce fragment est ligué dans l'ADN double brin du phage M13mp18 préalablement ouvert par Eco RI et HindIII, sites présents dans le site de liaisons multiples de ce vecteur. On transfecte alors des colonies de E. coli JM 101.

De l'analyse de colonies isolées, on retient le clone M13 EHCβ7 pour la suite, on purifie la forme simple brin de M13EHCβ7.

### b) Mutagénèse de Cβ2 de souris

La procédure utilisée est précisément décrite dans Maniatis et al. On doit cependant préciser les points suivants :
L'oligonucléotide de la figure 2 est synthétisé sur l'appareil Applied Biosystems 380B, puis purifié sur colonne sep Pak C18.

On hybride 0.2 µg du simple brin de M13EHCβ7 avec 3 pmoles de OhCβ kinase. Aucun autre oligonucléotide n'est utilisé. La température initiale de la réaction d'hybridation est 70° C.

La polymérase utilisée est la T4ADN polymérase (1 unité par réaction) pour compléter en double brin. La transfection est effectuée dans la souche E. coli JM101. La sélection des colonies mutantes se fait par hybridation avec l'oligonucléotide de la figure 2 radiomarqué. La température d'hybridation est 42° C. Deux lavages de 10 mn à 4° C ont été suffisants pour discriminer les colonies mutantes par autoradiographie.

L'ADN d'un sous-clone appelé muCβ215 d'une colonie repérée par hybridation radioactive a été digérée par BglII, puis séquencé par la méthode de Sanger pour confirmation de la mutation désirée et intégrité du reste de la séquence Cβ2 murine. L'ADN double brin de ce sous-clone est purifié et utilisé dans la suite des expériences.

### 3. Sous-clonage de la chaîne mutée dans p Bluescript SK⁺

p Bluescript SK⁺ est un plasmide entièrement caractérisé et disponible commercialement chez la société STRATAGENE.

L'ADN double brin de muCβ215 est coupé par EcoRI et EcoRV et le plus petit des deux fragments purifié sur gel d'Agarose. Ce fragment est ligué dans p Bluescript préalablement ouvert par EcoRI et SmaI, deux sites présents dans le site de liaisons multiples de ce plasmide, et transfecté dans la souche d'E. coli DH1 servant d'hôte à la chimère. Des colonies isolées sont analysées par coupure avec deux couples d'enzymes EcoRI-XbaI et EcoRI-BglII. Un clone, appelé pBSmuCβ215, fournit les bandes attendues (respectivement 1.2 kb et 0.5 kb environ) en électrophorèse. Ce plasmide est purifié et utilisé dans la suite des expériences.

### B) Obtention d'ADNc humain codant pour la chaîne du TCR

La technique de polymérisation en chaîne (PCR) est choisie pour les raisons citées ci-dessous :
- beaucoup de gènes codant pour la chaîne du TCR incluant différentes régions V sont publiés, dont le segment Vβ2,
- rapidité d'obtention d'ADNc par cette technique,
- sûreté d'avoir des gènes complets en 5′, comprenant le codon ATG d'initiation,
- facilité d'intégrer des sites de restriction en 5′ du gène pour faciliter le clonage.

Dans l'exemple préféré, et pour montrer la polyvalence permise par l'invention, plusieurs ADNc humains contenant différents segments 2 ont été isolés et couplés à la chaîne mutée Cβ2 de souris.

### - Obtention d'ARNm de cellules T humaines

On utilise un mélange de lymphocytes périphériques d'individus caucasiens obtenus auprès du Centre de Transfusion Sanguine de Marseille. Après Ficoll, les lymphocytes sont ajustés à 1 million de cellules par millilitre en milieu RPMI, 10 % sérum de veau foetal 20 mM glutamine, 1mM pyruvate de sodium, 500 UI de pénicilline, et stimulés à la phytohémagglutinine A (10 µg/millilitre de concentration finale) pendant 3 jours. 200 millions de cellules sont utilisées pour purifier leur RNA total.

Le protocole utilisé est décrit exactement dans Maniatis et al, pages 719 et suivantes. Environ 50 µg de RNA sont obtenus par cette méthode.

### 4 - Réactions de PCR

Un oligonucléotide OhCβ spécifique des deux chaînes humaines Cβ2 et Cβ1 et contenant le site unique BglII présent sur les segments géniques Cβ1 et Cβ2 est synthétisé de manière analogue à OmCβ.

Un oligonucléotide OL2 spécifique de la séquence de tête de Vβ2.1, Vβ2.2 et Vβ2.3 d'après la définition des sous-familles Vβ de Kimura et al (1987), Eur J Immunol ; 17 : 375-383 est également synthétisé.

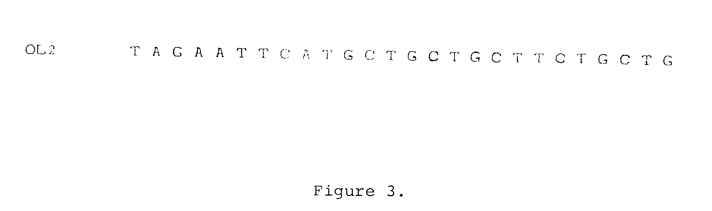

Cet oligonucléotide est présenté en figure 3.

Une réaction de PCR a été effectuée avec le couple d'oligonucléotides OhCβ-OL2.

La procédure expérimentale est décrite en détail dans Kawasaki et al (1989) PCR Technology, Principles and Applications for ADN Amplification ; Stockton Press, Henry. A Ehrlich Edition pp89-et suivantes. Les précisions suivantes sont utiles :
La quantité d'ARN de départ est de 1 µg pour la synthèse du brin complémentaire d'ADNc.

Pour la réaction de PCR proprement dite, 10 pmoles d'oligonucléotides OL2 et OhCβ, sont utilisés.

Le cycle de PCR est :
- 1 mn de dénaturation à 95° C,
- 1 mn d'hybridation à 52° C,
- 1 mn d'extension à 72° C,

et 30 cycles sont effectués sur le ADN Thermalcycler Version 2.2 de Perkin Elmer.

Le produit de la réaction est déposé sur gel d'Agarose

1.4 % pour électrophorèse. Après coloration au bromure d'éthidium, une bande distincte d'environ 500 pb est découpée et l'ADN électroélué. On obtient environ 30 ng d' ADN.

1 ng de cet ADN est amplifié une seconde fois par PCR. On purifie de la même manière que précédemment la bande spécifique et 300 ng d'ADN sont obtenus.

### C) Construction des gènes chimériques

### Principe:

On construit les gènes chimériques en clonant le fragment produit de PCR, coupé par EcoRI et BglII dans le grand fragment obtenu de la digestion de pBSmuCβ215.

On coupe pBSmuCβ215 par EcoRI et BglII et le grand fragment appelé FpBSmuCβ215 est purifié sur gel d'Agarose et électroélué.

100 ng du produit d'amplification obtenu avec les oligo-nucléotides OL2, OhCβ sont coupés par EcoRI et BglII.

On ligue ces deux fragments et le produit de la Iigation est transfecté dans DH1.

L'analyse de colonies isolées BSGβ2.1, ... BSGβ2.11 par coupure BglII-EcoRI fournit le fragment attendu d'environ 500 pb. Tous les sites sont séquencés entre le site EcoRI et le site BglII.

Les clones BSGβ2.7 et BSGβ2.11 sont retenus pour la suite des expériences. Ces deux clones possèdent la séquence d'acides aminés Vβ2.1 telle que décrite dans Toyonaga et al (déjà cité). Ils possèdent par contre des régions D et J différentes.

### EXEMPLE 2 : Expression des gènes chimériques

### Principe :

La chaîne du TCR est une protéine membranaire qui est exprimée à la surface si, et seulement si, les autres chaînes du complexe du TCR qui lui sont associées (ie chaîne α et complexe CD3) sont présentes.
Pour obtenir l'expression des gènes chimériques précédemment construits, on utilise la lignée cellulaire murine DOIS19 développée par Letourneur F et al (1989) Eur J Immunol : 19 : 2269-2274. DOIS19 est un mutant d'un hybridome T de souris qui possède tous les composants du TCR sauf la chaîne β. Cette lignée n'exprime donc pas de TCR à sa surface. La transfection d'une chaîne β exogène induit l'expression d'un récepteur fonctionnel dans les cellules tranfectées. Les deux gènes chimériques de l'invention contenus dans BSGβ2.7 et BSGβ2.11 ont été transfectés dans cette cellule suivant les protocoles décrits ci-après :

### 1) Sous-clonage des gènes chimériques dans un vecteur d'expression eucaryote

### Principe :

Le vecteur d'expression choisi est le plasmide pHβPr1-néo. Il est décrit précisément dans Gunning P et al (1987) Proc Natl Acad Sci USA ; 84 : 4831-4835. Le gène à exprimer est sous-cloné en aval du promoteur β actine et en amont d'un site de polyadénylation présents sur ce plasmide. Ces deux signaux assurent la synthèse d'un RNA messager fonctionnel du gène inséré. Par ailleurs, ce plasmide possède le gène de résistance néomycine qui confère la résistance à une série d'antibiotiques, dans l'exemple préféré l'antibiotique G418, aux cellules ayant intégré le plasmide.

De plus, une origine de réplication et un gène de résistance à l'ampicilline sont également présents permettant la sélection et la réplication dans E coli. La souche de E. coli utilisée est ici MC 1061.

### Expérimentation :

On coupe le plasmide BSGβ2.7 par les enzymes Bam HI et Sal I et le plus petit des deux fragments (1.2 kb) est purifié sur gel d'Agarose. Ce fragment comportant la totalité de la séquence codante du gène chimérique est ligué au plasmide pHβPr1-néo préalablement ouvert par Bam HI et Sal I. Le produit de ligation est transfecté dans E. coli MC 1061 et de l'analyse de colonies isolées par la coupure BamHI et SalI, on retient le clone appelé néo Gβ2.7 qui fournit le fragment de 1.2 kb attendu sur l'électrophorèse d'Agarose.

De la même façon, le gène chimérique contenu dans pBSGβ2.11 est sous-cloné dans pHβPrl-néo et le clone appelé néo Gβ2.11 est conservé. Néo Gβ2.7 a été déposé à la CNCM le 30 avril 1991 sous le n° I - 1090.

### 2. Transfection des gènes chimériques dans DOIS19

La procédure de transfection choisie est l'électroporation. Les plasmides néo Gβ2.7 et néo Gβ2.11 sont purifiés sur gradient de chlorure de Césium tel que décrit dans Maniatis et al (déjà cité), et repris dans du TE (Tris 10 mM, EDTA 1 mM pH8), à raison de 1 mg/ml. Les cellules DOIS19 sont cultivées en milieu complet (Dubelco modified Eagle Médium, (DMEM) 10 % de serum veau foetal (SVF), 2 mM glutamine, 1 mM sodium pyruvate, 500 IU penicilline, 500 IG streptomycine, 50 µM β mercaptoéthanol). 10 millions de cellules DOIS19 en phase exponentielle de croissance sont lavées 3 fois en tampon phosphate salé (PBS : 0,14 M NaCl, 10 mM phosphate de sodium pH 7,2). Les cellules sont reprises dans 500 µl de PBS dans une cuve d'électroporation (Biorad), 0,4 cm entre les électrodes. On ajoute 10 µl de plasmide à transfecter (10 µg) et on incube 10 mn à température ambiante. Le choc électrique est effectué conformément aux instructions du constructeur sur un appareil Biorad (Gene Pulser Apparatus) suivant les conditions suivantes : voltage à 250 V, capacité à 250 micro Farad. Après le choc, les cellules sont incubées 10 mn à température ambiante puis reprise en milieu complet. Les cellules sont ensuite incubées 24 h à 37° C en atmosphère humide à 7% CO₂. On distribue alors les cellules à raison de 5. 10⁴ cellules par ml en plaques 24 puits (Falcon) en milieu complet supplémenté à 3 mg/ml d'antibiotiques G418 (GIBCO).

Après 15 jours les colonies résistantes à l'antibiotique sont analysées en cytométrie de flux. Avec le plasmide néo Gβ2.7 on obtient les clones résistants FRN2.7.1 ... FRN2.7.24. Avec le plasmide néo Gβ2.11 on obtient les clones FRN2.11.1 ... FRN2.11.24. Ces clones sont analysés en cytométrie de flux avec un anticorps anti CD3 murin (anticorps 2C11 décrit dans Leo et (1987) Proc Natl Acad Sci USA 84 : 1374).

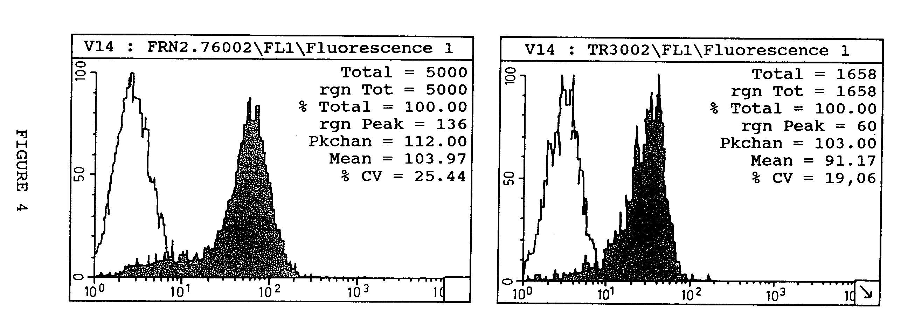

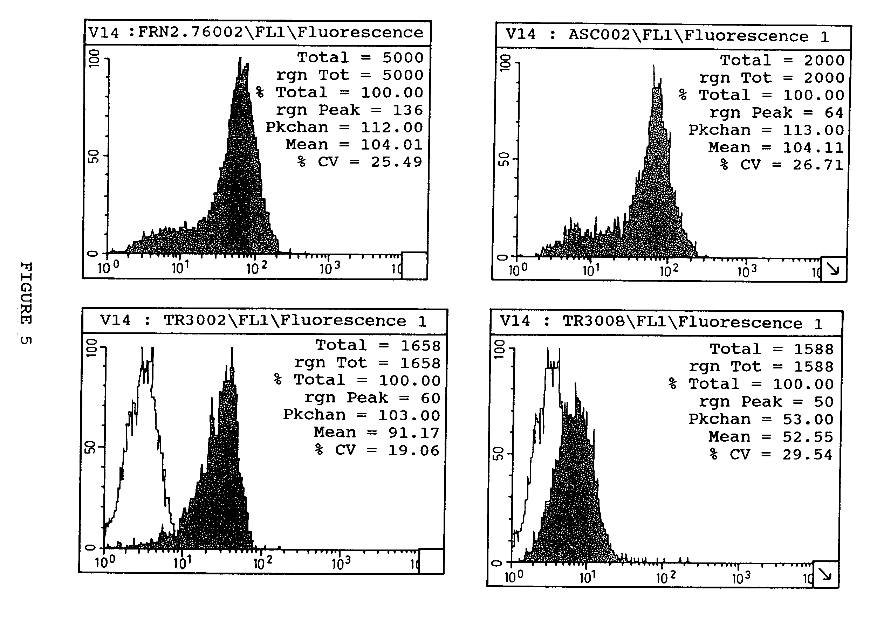

Pour chaque clone, 10⁵ cellules dans une solution de DMEM 4% SVF, 0,02% azide de sodium (milieu de FACS) et 15 µg/ml d'anticorps anti CD3 murin sont incubés pendant 45 mn à 4° C sous agitation. Après trois lavages en milieu de FACS, on incube les cellules avec un anticorps polyclonal de chèvre anti-immunoglobuline de rat marqué au FITC (Réactit Immunotech à 15 µg/ml en milieu de FACS pendant 30 mn à 4° C sous agitation. Après 3 lavages en milieu de FACS, les cellules sont fixées en PBS à 1% formaldéhyde et analysées sur l'appareil de cytométrie de flux Facscan de Becton Dickinson. 10⁵ cellules DOIS19 sont traitées de la même façon et servent de contrôle négatif. Les profils obtenus pour les deux meilleurs expresseurs de chacune des transfections (FRN2.7.6 pour la première et FRN2.11.2 pour la seconde) sont montrés en figure 4 (profils noirs) par rapport au profil de DOIS19 (profil blanc). La lignée cellulaire FRN2.7.6 a été retenue pour l'immunisation.

### 3. Immunisation des souris Balb/c

Trois souris Balb/c sont immunisées avec FRN2.7.6. Le protocole d'immunisation est le suivant :
- Jour 0 : 10 millions de cellules FRN2.7.6 injectées par voie intrapéritonéale.
- Jour 20 : 10 millions de cellules FRN2.7.6 injectées par voie intrapéritonéale.
- Jour 40 : 5 millions de cellules injectées par voie intraveineuse.
- Jour 43 : Prélèvement des rates pour fusion.

### 4. Fusion par hybridation lymphocytaire

On fusionne les splénocytes de deux souris immunisées avec le myélome X63 Ag 8.653 suivant le protocole décrit par Kohler et Milstein (1975) Nature 256 ; 495-497.

L'analyse des surnageants des clones résultant de la fusion se fait par cytométrie de flux sur FRN2.7.6 versus DOIS19.

100 µl de surnageant de chaque clone sont prélevés.50 µl sont incubés avec 50 µl de milieu de FACS contenant 10⁵ cellules FRN2.7.6. Les 50 µl restant sont incubés avec 50 µl de milieu de FACS mais contenant 10⁵ cellules DOIS19. Le protocole est le même par ailleurs que celui utilisé pour l'analyse des transfectants.

Des 300 surnageants testés, deux surnageants marquent FRN2.7.6 et ne marquent pas DOIS19. Les deux monoclonaux correspondant E25G8 et E22E7 sont analysés de manière plus poussée.

### 5. Analyse des deux monoclonaux E25G8 et E22E7

### a. Cytométrie de flux sur les transfectants FRN2.7.6 et FRN2.11.2

La figure 5 montre les profils obtenus sur les deux transfectants FRN2.7.6 et FRN2.11.2 et DOIS19 avec les surnageants E25G8 et E22E7 suivant le protocole décrit pour le screening des anticorps comparés aux profils obtenus avec l'anti CD3 murin. On constate que E22E7 marque de façon comparable par rapport à l'anti CD3 les trois lignées FRN2.7.6, FRN2.11.2 et DOIS19. Par contre, E25G8 marque beaucoup moins bien FRN2.11.2 par rapport à l'anti CD3.

### b. Cytométrie de flux sur des transfectants portant d'autres Vβ humains

De la même manière que pour les transfectants FRN2.7.6 et FRN2.11.2, on a obtenu des transfectants FRN8.1.2 exprimant un gène chimère portant Vβ8.1 ; FRN17.4.2 exprimant un gène chimère portant Vβ17.1 en transfectant les gènes chimères correspondant dans DOIS19.

Les deux surnageants E22E7 et E25G8 ne marquent pas ces différentes lignées.

### c. Cytométrie de flux sur sang total

Si les anticorps reconnaissent bien la chaîne Vβ2, une sous population de lymphocytes T périphériques exprimant Vβ2 doit être marquée spécifiquement par ces anticorps. Des doubles marquages ont été effectués sur sang périphérique avec les anticorps E22E7 et E25G8 révélés par un polyclonal marqué au FITC, et l'anticorps SPVT3b spécifique du CD3 humain (réactif Immunotech) marqué à la phicoérythrine.

### Expérimentation

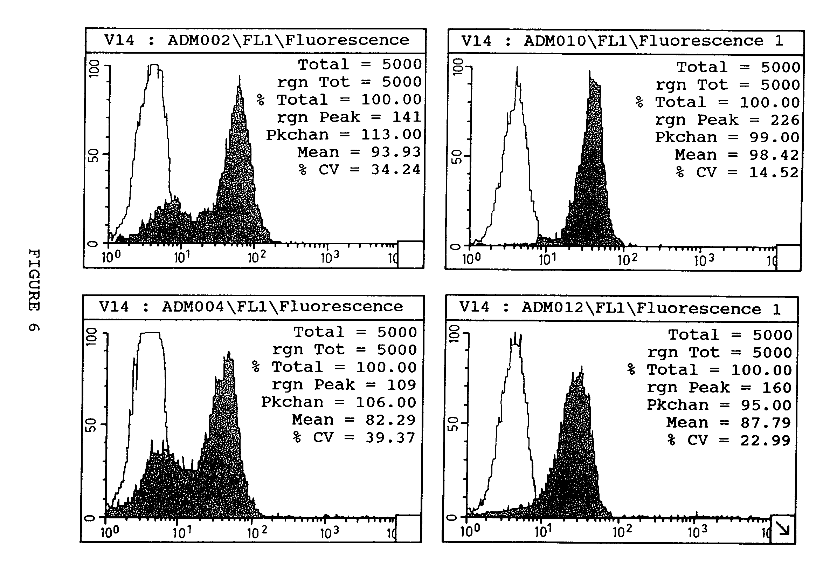

Le sang est prélevé sur EDTA (10 mM final) pour éviter la coagulation. 100 µl de sang sont centrifugés et le culot cellulaire est repris par 200 µl de surnageant E22E7 et incubé 30 mn à température ambiante. Deux lavages sont effectués en milieu de FACS et le culot cellulaire est repris par 100 µl de milieu de FACS contenant 15 µg/ml d'un anticorps polyclonal de chèvre anti-immunoglobuline de souris (réactif Immunotech). Deux lavages sont effectués en milieu de FACS. Le culot cellulaire est repris en milieu de FACS contenant 15 µg/ml d'un anticorps de souris distinct (réactif de contrôle IgG2a d'Immunotech). On incube 2 mn à température ambiante. On effectue deux lavages supplémentaires en milieu de FACS et le culot cellulaire est repris dans 200 µl de milieu de FACS contenant 10 µg/ml de l'anticorps SPVT3b marqué à la phycoérythrine (réactif Immunotech). On incube 15 mn à température ambiante. On effectue deux lavages en milieu de FACS. On ajoute 1 ml de tampon de lyse préchauffé à 25° C (dilution 1/25 du réactif de lyse d'Immunotech) et l'on mélange vivement aussitôt. Après la lyse, on ajoute 250 µl de fixateur (réactif Immunotech). On centrifuge et on reprend le culot dans 0,5 ml de PBS à 0,5% de formaldéhyde. Les cellules sont analysées sur Facscan de Becton Dikckinson. Le résultat obtenu avec le surnageant E22E7 est présenté en figure 6. On constate qu'environ 5% des cellules T sont marquées par l'anticorps E22E7. La même manipulation effectuée avec l'anticorps E25G8 ne donne pas de marquage significatif d'une sous population T.

Par le même protocole, on effectue des marquages sur le sang d'autres individus à l'aide de l'anticorps E22E7. Cet anticorps marque entre 2 et 10% des cellules T périphériques pour les individus testés.
On en déduit, en raison de l'homologie de séquence supérieure à 99 % entre les différents segments Vβ2 humains, que l'anticorps F22E7 est spécifique des segments Vβ2, notamment Vβ2.1.

L'anticorps E25G8 reconnaît soit la jonction VDJ du clone néo Gβ2.7, soit l'association α souris, β humain.

### 5. Intérêt de l'anticorps E22E7 dans le choc septique

Les analyses en PCR on montré l'expansion du compartiment TVβ2 positif lors du choc septique à la toxine de staphylocoque TSST (Choï Y et al (1990) Exp Med 172 : 981-984). Des études de répertoires, lors de tels chocs, sont grandement simplifiées par l'usage d'anticorps monoclonaux anti Vβ2. L'intérêt de l'anticorps de l'invention, E22E7, lors de ces chocs, ressort du test in vitro ci-après montrant la mise en évidence d'une expansion spécifique du compartiment Vβ2 par la toxine TSST par l'anticorps E22E7.

### Expérimentation

On prélève stérilement du sang d'un individu sain. Après Ficoll, les cellules blanches du sang sont distribuées en milieu complet contenant soit 1µg/ml TSST (Toxin Technology), soit 10 µg/ml phytohémagglutinine A (PHA) (Sigma) à 5.10⁵ cellules par millilitre en petit flacon de culture (Falcon).

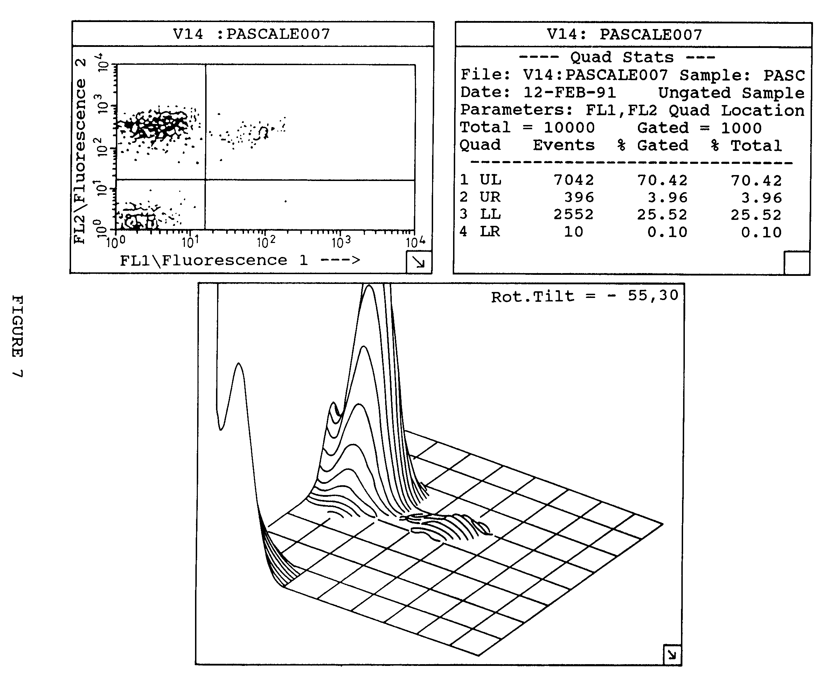

Après 72 h de culture, les cellules sont lavées en milieu complet et reprises à 10⁶ cellules par millilitre en milieu complet contenant 5 U/ml d'interleukine 2 (Cetus) en plaques 24 puits. Après 24 h, les cellules sont analysées en cytométrie de flux avec un anticorps distinct (contrôle négatif) anti Vβ2 de souris, un anticorps CD3 humain (SPVT3b, anticorps Immunotech), et E22E7, anticorps de l'invention. Ces anticorps sont révélés avec un anticorps polyclonal de chèvre anti souris. marqué au FITC. La figure 7 montre l'expansion spécifique du compartiment des cellules Vβ2 positives lorsque de la stimulation à la TSST par rapport à l'activation par la PHA qui active toutes les cellules T non spécifiquement.

### EXEMPLE 3

On a préparé une préparation injectable comprenant :

| | |
|---|---|
| - Anticorps monoclonaux de l'exemple 2 | 5 mg |
| - eau pour préparations injectables | 1 ml |

## Revendications

1. ADN recombinant caractérisé en ce qu'il comprend une séquence nucléotidique chimère codant pour la chaîne β du récepteur pour l'antigène des cellules T, ladite séquence étant constituée :
- d'une séquence d'origine humaine et codant pour la totalité d'une partie variable (Vβ2) et sa séquence de tête, diversité (Dβ) et de jonction (Jβ),
- et d'une séquence d'origine murine codant pour la partie complémentaire constante (Cβ1 ou Cβ2) absente de la séquence humaine ci-dessus,
les deux séquences étant liées au niveau d'un site de restriction naturel ou créé artificiellement.

2. ADN recombinant selon la revendication 1 caractérisé en ce que le site de restriction est naturel sur la séquence codant pour la partie Cβ.

3. ADN recombinant selon la revendication 1 ou 2 caractérisé en ce que le site de restriction est BglII.

4. ADN recombinant selon l'une quelconque des revendications 1 et 3 caractérisé en ce que le site de restriction est créé artificiellement sur la chaîne d'origine murine.

5. ADN recombinant selon l'une quelconque des revendications 1 à 4 caractérisé en ce qu'il s'agit d'un vecteur d'expression eucaryote.

6. ADN recombinant selon la revendication 5 caractérisé en ce que le vecteur d'expression eucaryote est un plasmide dérivant de PhβPr1-Néo.

7. Anticorps caractérisés en ce pou'ils sont obtenus par une succession d'étapes dans lesquelles intervient un ADN recombinant selon l'une quelconque des revendications 1 à 6.

8. Anticorps anti chaîne Vβ2 du récepteur des cellules T humaines.

9. Compositions pharmaceutiques caractérisées en ce qu'elles renferment des anticorps selon la revendication 7 ou 8.

10. Produits de diagnostic caractérisés en ce qu'ils renferment des anticorps selon la revendication 7 ou 8.

11. Procédé de préparation d'un ADN recombinant tel que défini à la revendication 1, caractérisé en ce que l'on ligue :
- une séquence nucléotidique constituée d'au moins la totalité de la séquence codant pour les parties Vβ2, Dβ et Jβ d'une chaîne du récepteur de l'antigène des cellules T humain jusqu'au niveau d'un site de restriction, avec
- une séquence nucléotidique d'origine murine comprenant l'homologue de la partie complémentaire de la partie Cβ absente de la séquence de chaîne humaine ci-dessus, à partir du même site de restriction que ci-dessus,
ledit site de restriction étant artificiellement créé sur l'une, l'autre ou les deux séquences, pour obtenir la chimère attendue, puis si désiré, ligue une séquence comprenant la totalité des parties Vβ2, Dβ, Jβ et Cβ ci-dessus avec un vecteur d'expression eucaryote, puis si désiré, isole la nouvelle chimère obtenue.

12. Procédé de préparation d'anticorps mettant en oeuvre initialement le procédé selon la revendication 11 qui comporte une étape de préparation de l'ADN recombinant défini à la revendication 5 caractérisé en ce qu'en outre l'on transfecte une cellule eucaryote comportant tous les éléments permettant la synthèse du récepteur de l'antigène des cellules T, à l'exception de la chaîne homologue à la chaîne codée par la séquence d'ADN chimère ci-dessus, utilise comme antigène les cellules ainsi transfectées chez la race murine correspondant, fusionne des cellules de rate prélevées sur les murins auxquels a été administré l'antigène à des cellules de myélome pour préparer, selon les méthodes connues en elles-mêmes, des anticorps et sélectionne les anticorps contre le produit de la séquence variable humaine à l'aide des cellules eucaryotes transfectées à l'aide des ADN chimères ci-dessus.


INFORMATION pour SEQ ID NO 3 (fig. 1) :

(i) CARACTERISTIQUES DE LA SEQUENCE :
(A) LONGUEUR : 519 paires de bases
(B) TYPE : acide nucléique
(C) NOMBRE DE BRINS : simple brin
(D) CONFIGURATION : linéaire

(ii) TYPE DE MOLECULE : cADN
(A) Origine : murine

(xi) DESCRIPTION DE LA SEQUENCE : SEQ ID NO 3 :

GAGGATCTGA GAAATGTGAC TCCACCCAAG GTCTCCTTGT TTGAGCCATC AAAAGCAGAG 60

ATTGCAAACA AACAAAAGGC TACCCTCGTG TGCTTGGCCA GGGGCTTCTT CCCTGACCAC 120

GTGGAGCTGA GCTGGTGGGT GAATGGCAAG GAGGTCCACA GTGGGGTCAG CACGGACCCT 180

CAGGCCTACA AGGAGAGCAA TTATAGCTAC TGCCTGAGCA GCCGCCTGAG GGTCTCTGCT 240

ACCTTCTGGC ACAATCCTCG AAACCACTTC CGCTGCCAAG TGCAGTTCCA TGGGCTTTCA 300

GAGGAGGACA AGTGGCCAGA GGGCTCACCC AAACCTGTCA CACAGAACAT CAGTGCAGAG 360

GCCTGGGGCC GAGCAGACTG TGGAATCACT TCAGCATCCT ATCATCAGGG GGTTCTGTCT 420

GCAACCATCC TCTATGAGAT CCTACTGGGG AAGGCCACCC TATATGCTGT GCTGGTCAGT 480

GGCCTGGTGC TGATGGCCAT GGTCAAGAAA AAAAATTCC 519

INFORMATION pour SEQ ID NO 4 (fig. 1) :

(i) CARACTERISTIQUES DE LA SEQUENCE :
(A) LONGUEUR : 531 paires de bases
(B) TYPE : acide nucléique
(C) NOMBRE DE BRINS : simple brin
(D) CONFIGURATION : linéaire

(ii) TYPE DE MOLECULE : cADN
(A) Origine : humaine

(xi) DESCRIPTION DE LA SEQUENCE : SEQ ID NO 4 :

GAGGACCTGA ACAAGGTGTT CCCACCCGAG GTCGCTGTGT TTGAGCCATC AGAAGCAGAG 60

ATCTCCCACA CCCAAAAGGC CACACTGGTG TGCCTGGCCA CAGGCTTCTT CCCCGACCAC 120

GTGGAGCTGA GCTGGTGGGT GAATGGGAAG GAGGTGCACA GTGGGGTCAG CACAGACCCG 180

CAGCCCCTCA AGGAGCAGCC CGCCCTCAAT GACTCCAGAT ACTGCCTGAG CAGCCGCCTG 240
AGGGTCTCGG CCACCTTCTG GCAGAACCCC CGCAACCACT TCCGCTGTCA AGTCCAGTTC 300
TACGGGCTCT CGGAGAATGA CGAGTGGACC CAGGATAGGG CCAAACCCGT CACCCAGATC 360
GTCAGCGCCG AGGCCTGGGG TAGAGCAGAC TGTGGCTTTA CCTCGGTGTC CTACCAGCAA 420
GGGGTCCTGT CTGCCACCAT CCTCTATGAG ATCCTGCTAG GGAAGGCCAC CCTGTATGCT 480
GTGCTGGTCA GCGCCCTTGT GTTGATGGCC ATGGTCAAGA GAAAGGATTT C 531